(19) Europäisches Patentamt / European Patent Office / Office européen des brevets

(11) **EP 3 822 615 B1**

(12) **EUROPEAN PATENT SPECIFICATION**

(45) Date of publication and mention
of the grant of the patent:
**03.04.2024 Bulletin 2024/14**

(21) Application number: **20208329.1**

(22) Date of filing: **18.11.2020**

(51) International Patent Classification (IPC):
*G01N 15/08* (2006.01)    *G01N 1/40* (2006.01)

(52) Cooperative Patent Classification (CPC):
**G01N 15/08; C12H 1/063; G01N 33/146;**
G01N 15/0826; G01N 2001/4088; G01N 2015/084

(54) **EQUIPMENT AND PROCESS FOR EXECUTING FILTRATION TESTS ON A LIQUID**

VORRICHTUNG UND VERFAHREN ZUR DURCHFÜHRUNG VON FILTRATIONSTESTS AN EINER FLÜSSIGKEIT

ÉQUIPEMENT ET PROCÉDÉ D'EXÉCUTION DE TESTS DE FILTRATION SUR UN LIQUIDE

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **18.11.2019 IT 201900021468**

(43) Date of publication of application:
**19.05.2021 Bulletin 2021/20**

(73) Proprietor: **WIN&TECH S.R.L.**
**37024 Negrar (VR) (IT)**

(72) Inventor: **BOIN, Stefano**
**35020 Albignasego (PD) (IT)**

(74) Representative: **Caldon, Giuliano et al**
**Gallo & Partners S.r.l.**
**Via Rezzonico, 6**
**35131 Padova (IT)**

(56) References cited:
DE-A1- 19 831 946      DE-A1- 19 831 947
US-A1- 2019 001 246

• **Paul Bowyer ET AL: "NTU vs wine filterability index - what does it mean for you?", Grapegrower and Winemaker Issue 585, 2012 / Blue H2O Filtration, 31 January 2015 (2015-01-31), page 76, XP055713184,** https://winetitles.com.au/gwm/articles/october-585/ntu-vs-wine-filterability-what-does-it-mean-for-you/ **Retrieved from the Internet: URL:https://bhftechnologies.com.au/wp-content/uploads/2015/01/Article-NTU-vs-Wine-Filterability-Index-What-Does-It-Mean-For-You-updated-0115.pdf [retrieved on 2020-07-09]**
• **Oenolab Diagnostics: "OENOLAB FILTER TEST UNIT - DETERMINATION OF THE WINE FILTERABILITY INDEX", , 26 March 2018 (2018-03-26), XP055713192, Retrieved from the Internet: URL:https://www.oenolab.com/en/filtrability/28-ftu.html [retrieved on 2020-07-09]**
• **Exacta-Optech: "Filterability tester A11327", , 23 January 2018 (2018-01-23), XP055713176, Retrieved from the Internet: URL:https://www.exactaoptech.com/enologia/test-filtrabilita/indice-di-filtrabilita/ [retrieved on 2020-07-09]**
• **Gusmer Enterprises: "Wine Preparation", , 29 April 2013 (2013-04-29), XP055713168, Retrieved from the Internet: URL:https://www.gusmerwine.com/wp-content/uploads/sites/7/2015/12/wine-preparation-and-hose.pdf [retrieved on 2020-07-09]**

EP 3 822 615 B1

**Description**

Field of application

The present invention regards equipment and a process for executing filtration tests on a liquid, according to the preamble of the relative main independent claims.

The equipment and the process, object of the present invention, are intended to be advantageously used for determining filtration parameters, such as the filterability index (IF), the modified filterability index (IFM), the maximum filterable volume (Vmax) and the Silt Density Index (SDI).

The equipment and the process, object of the present invention, are therefore advantageously intended to be used on liquids of different type, intended to be filtered during the processing thereof, in particular liquid foods such as water, wine, beer, syrups, oil and other drinks.

The present equipment and process are therefore inserted in the industrial field of production of laboratory instruments, in particular for the analysis of filtration parameters associated with liquids and membranes.

State of the art

In the field of production of laboratory instruments, equipment pieces are known for executing filtration tests on liquids, which are intended to determine several filtration parameters associated with such liquids and with the filters with which these are intended to be filtered.

For example, such equipment pieces are widely used in the winemaking field for determining the so-called filterability index (IF) of a wine or of a must, which is aimed to indicate if the wine (or the must) is suitable or unsuitable for being filtered by means of a particular filter, for example before bottling. In addition, such equipment pieces are advantageously used also for determining the micrometry of the filter most suitable for filtering the aforesaid wine (or must), in a manner such to optimize the filtration process.

For such purpose, equipment pieces are generally known that are provided with a first container, generally having small dimensions, which is filled with the liquid to be analyzed, and with a measurement duct, which is hydraulically connected on one side to the first tank and on the other side to a filter to be tested. Downstream of the filter, a dispensing spout is also generally present, closed by means of a valve, and downstream of such dispensing spout a second graduated container is generally placed.

In operation, the filtration test thus provides for placing first container under pressure, in particular at a pressure that is constant over time and generally equal to 2 bar, and subsequently opening the valve of the dispensing spout, collecting the filtered liquid in the second graduated container. In addition, during the filtration test, the time instants are identified at which the filtered liquid reaches specific levels of the graduated container, generally equal to 200 ml, 400 ml and 600 ml.

Alternatively with respect to the second graduated container, it is also known to place the second container on a scale in order to determine the mass of the filtered liquid and, knowing the density of the liquid, calculate the volume thereof.

On the basis of formulas that are well-known and standardized in the field, starting from the measurements of time collected during the analysis, the filterability index (IF) associated with the tested liquid and with the particular tested filter is calculated.

In addition, on the basis of the data collected during the filterability test, it is also known to calculate further parameters, which allow better describing the characteristics of filterability of the liquid. In particular, such parameters are the modified filterability index (IFM), the maximum filterable volume (Vmax) and the Silt Density Index (SDI), which is generally used for the filtration of water.

In the latter case, the filterability test requires quantities of liquid greater than those requested in order to determine the filterability index (IF). In operation, indeed, in order to obtain the SDI it is necessary to measure an initial time corresponding to the filtration of 0.5 liters of water, subsequently leaving the equipment to work for an intermediate time period, generally 15 minutes, and finally newly measuring a final time corresponding to the filtration of an additional 0.5 liters of water.

The equipment pieces for executing filterability tests of known type briefly described up to now have in practice shown that they do not lack drawbacks.

A first drawback of such equipment pieces of known type lies in the small capacity of the first and second container containing the liquid to be tested and the liquid filtered during the test. Indeed, such containers are generally provided with limited capacities in order to avoid pressurizing large quantities of liquid, and thus obtain equipment pieces that are simpler and less expensive.

Such sizing of the containers however involves the drawback of not being able to execute filterability tests on large quantities of liquid. In particular, such drawback is particularly present for measuring the SDI, since the quantities of liquid necessary for executing such measurement are much greater than those necessary for the other tests of known type.

A further drawback of the equipment pieces of the above-described known type lies in the high incidence of random errors present in the tests carried out, which are introduced by the operator who carries out the tests themselves. Indeed, the tests carried out by means of such equipment pieces of known type require the operator to execute detections in a manual manner, thus introducing errors that affect the calculation of the filtration parameters.

A further drawback of the equipment pieces of known type lies in the fact that these are not very practical in use and the filtration tests require rather long attainment times. Indeed, the operations to be executed during such test, such as the filling and pressurizing of the first container, the emptying of the second graduated container, the calibration of the scale (when present), can be rather long and/or complex in execution. In addition, the operations of pressurizing and calibration must be repeated each time it is necessary to newly fill the first tank or repeat the test (even repeating the test on the same liquid).

US 2019/001246 A1 shows a filterability tester for oil products having only one filter parameter evaluated, according to the preamble of the independent claims.

DE 198 31 946 A1 shows a wine filterability tester, using only a fluid amount as a filter parameter.

Presentation of the invention

In this situation, the problem underlying the present invention is therefore that of eliminating the problems of the abovementioned prior art, by providing equipment and a process for executing filtration tests on a liquid which allow being applied also to large quantities of liquid to be tested.

A further object of the present invention is to provide equipment and a process which allow preventing the use of containers under pressure.

A further object of the present invention is to provide equipment and a process which allow reducing the errors deriving from the operator who executes the test.

A further object of the present invention is to provide equipment and a process that are versatile.

A further object of the present invention is to provide equipment that is simple and inexpensive to make.

A further object of the present invention is to provide a process that is quick, efficient and easy to actuate.

Brief description of the drawings

The technical characteristics of the invention, according to the aforesaid objects, can be clearly seen in the contents of the below-reported claims and the advantages thereof will be more evident in the following detailed description, made with reference to the enclosed drawings, which represent a merely exemplifying and non-limiting embodiment of the invention, in which:

- figure 1 shows a diagram relative to one embodiment of the equipment for executing filtration tests on a liquid, object of the invention;
- figure 2 shows an exemplifying block diagram of a logic control unit of the equipment of figure 1.

Detailed description of a preferred embodiment

With reference to the enclosed drawings, reference number 1 overall indicates equipment for executing filtration tests on a liquid in accordance with the present invention.

The equipment 1 is advantageously intended to be used in the industrial field of treating liquids, in particular in laboratories and industries involved with the treatment and bottling of liquid foods, such as water, wine, beer, syrups, oil and drinks of another kind. Of course, the equipment 1 can also be used in other industrial fields, involved with treating non-liquid foods.

In particular, the present equipment 1 allows executing filtration tests and consequently determining filtration parameters F so as to optimize the industrial processes for treating liquids.

Hereinbelow with the expression "filtration parameter" it will be intended any type of parameter indicating the filterability of a liquid to be tested L, i.e. of its filling capacity in relation to a specific filter, as known in the reference field.

Therefore, the equipment 1 is intended to be advantageously used for determining parameters such as the filterability index (IF), the modified filterability index (IFM), the maximum filterable volume (Vmax), and the Silt Density Index (SDI). Advantageously, in addition, the equipment 1 is also usable for determining, in an empirical manner, the filter most suitable for executing a filtration treatment of the liquid to be tested L.

For such purpose, the equipment 1 is susceptible of being associated with a first container 2 containing the liquid to be tested L, in particular in a manner such to be fed with such liquid to be tested L. For example, the first container 2 is advantageously a laboratory cylinder, or a tank, or a piping, or any other type of industrial or laboratory container susceptible of containing the liquid to be tested L in a quantity sufficient for executing the filtration test.

In addition, the equipment 1, in order to be associated with the first container 2, can advantageously be at least partially inserted within the first container 2 and preferably it is fixed to a wall of the first container 2 itself, or associated thereto by means of a process that allows supplying the equipment 1 with the liquid to be tested L, contained in the first container 2.

According to the invention, the equipment 1 comprises a measurement duct 3, which is extended between an inlet section 31 and an outlet section 32, which are suitably open in order to allow the passage of the liquid to be tested L through the measurement duct 3 itself. According to the invention, moreover, the inlet section 31 is in fluid communication with the first container 2, in order to receive the liquid to be tested L, and preferably the outlet section 32 is in fluid communication with a second container 8, suitably arranged in order to receive the liquid to be tested L exiting from the measurement duct 3.

Advantageously, the equipment 1 is provided with a supporting tray, not illustrated in the enclosed figures, susceptible of carrying the first and the second container 2, 8 in a supported manner in order to retain them in position during the steps of a process for executing filtration tests, in particular of the type described hereinbelow.

Of course, without departing from the protective scope of the invention, the equipment 1 can also lack the supporting tray. In such case, the equipment 1 is preferably provided with connection means for mechanically connecting the measurement duct 3 at least to the first container 2 and preferably also to the second container 8. In particular, such connection means are preferable with respect to the supporting tray if the containers 2, 8 are provided with large dimensions, such as in the case of industrial tanks.

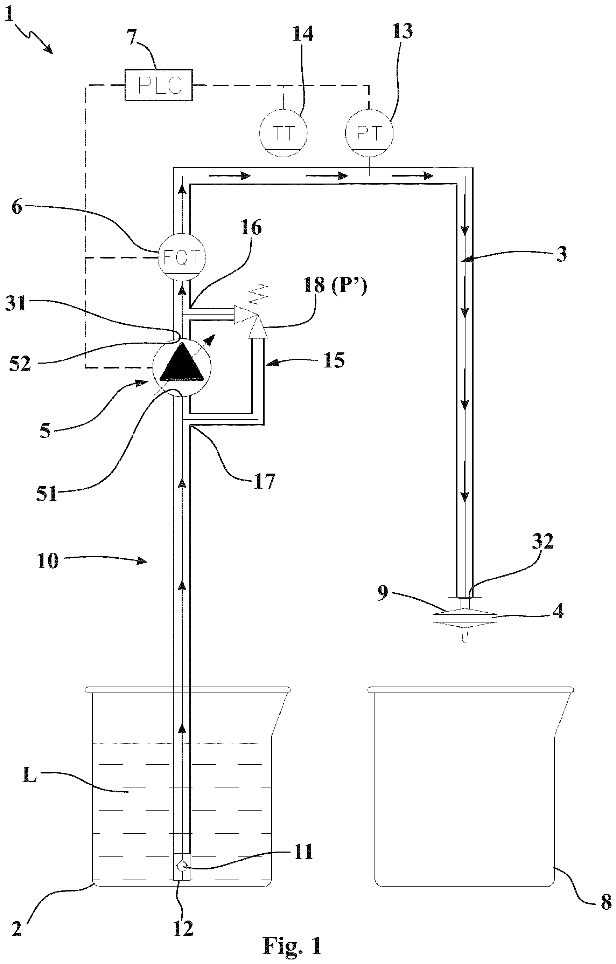

According to the invention, the equipment 1 is also provided with a filter 4 hydraulically connected to the measurement duct 3 and adapted to filter the liquid to be tested L. Preferably, moreover, the filter 4 is connected to the outlet section 32 of the measurement duct 3, as illustrated in the enclosed figure 1.

Advantageously, the equipment 1 also comprises a containment body 9 placed to intercept the measurement duct 3, preferably at the outlet section 32 thereof, and adapted to contain the filter 4. In particular, the containment body 9 is advantageously a hollow body, and preferably a membrane-holder container, at whose interior the filter 4 is susceptible of being housed.

In accordance with the enclosed figure 1, the filter 4 is irremovably fixed within the containment body 9, which in turn is mounted via fitting or screwing on the outlet section 32 of the measurement duct 3. In operation, therefore, in order to substitute the filter 4, the containment body 9 is removed and substituted with a different containment body 9 containing a different filter 4 at its interior.

Otherwise, according to a non-illustrated embodiment variant, it is also possible that the filter 4 be removably fixed within the containment body 9. In such configuration, the containment body 9 is advantageously made of two half-shells, susceptible of being constrained to each other to contain the filter 4 and of being separated from each other in order to substitute the filter 4 fixed at their interior. In addition, only one of such half-shells is mounted on the outlet section 32 of the measurement duct 3, for example via fitting or screwing, in order to receive the liquid to be tested L.

Otherwise, according to a further embodiment variant, the containment body 9 is susceptible of being placed to intercept the measurement duct 3, in a position upstream with respect to the outlet section 32. In particular, the containment body 9 is substantially configured as a slide valve, and is susceptible of being inserted and extracted from the measurement duct 3 itself, for example by sliding along suitable guides arranged inside the measurement duct 3. In such case, the containment body 9 can therefore be moved between a closed configuration, in which it is inserted within the measurement duct 3, and an open configuration, in which it is extracted from the measurement duct 3, for the purpose of allowing the insertion, the removal and the substitution of the filter 4. Advantageously, moreover, in the closed configuration the containment body 9 is hermetically sealed with the measurement duct 3 in order to prevent the leakage of liquid to be tested L from the equipment 1.

In accordance with the idea underlying the present invention, the equipment 1 also comprises a suction pump 5, which is hydraulically connected to the inlet section 31 of the measurement duct 3 and is adapted to suction the liquid to be tested L from the first container 2 and to convey it into the measurement duct 3.

In particular, the suction pump 5 is provided with a suction section 51 and with a delivery section 52, with the latter advantageously hydraulically connected to the inlet section 31 of the measurement duct 3.

The suction pump 5 is preferably of volumetric type, in particular with gears. Advantageously, moreover, the suction pump 5 is intended to be placed above head, i.e. at a height greater than the height of the surface of the liquid to be tested L contained within the first container 2.

Advantageously, the equipment 1 also comprises a suction duct 10, which is interposed between the suction section 51 of the suction pump 5 and the first container 2, in order to hydraulically connect the suction pump 5 with such first container 2.

In particular, as illustrated in the enclosed figure 1, the suction duct 10 is provided with an end section 12 susceptible of being immersed in the first container 2 and is preferably provided with a check valve 11 placed at such end section 12 in order to prevent the return of the liquid to be tested L within the first container 2 with the suction pump 4 non-operative.

In accordance with a different embodiment, not illustrated in the enclosed figures, the suction pump 5 can be directly connected to the first container 2 through the suction section 51 thereof. In such case, the suction pump 5 is preferably of submerged type. According to the idea underlying the present invention, the equipment 1 also comprises flow rate measurement means 6 placed to intercept the measurement duct 3 and preferably, in accordance with the enclosed figure 1, the flow rate measurement means 6 are placed upstream of the filter 4 with respect to the movement sense of the liquid to be tested L within the measurement duct 3.

The flow rate measurement means 6 are adapted to detect the flow rate of the liquid to be tested L which flows in the measurement duct 3 and to generate a plurality of flow rate signals Q, corresponding to the detected flow rate measurements. In particular, such flow rate measurement means 6 are of thermal dissipation type, per se known in the field and thus not described in detail hereinbelow.

In particular, in accordance with a preferred embodiment of the equipment 1, each flow rate signal Q generated by the flow rate measurement means 6 corresponds with the passage of a preset volume V of liquid to be tested L through the measurement duct 3. For example, the flow rate measurement means 6 are advantageously configured for generating a flow rate signal Q at each passage of 5 ml of liquid to be tested L. In such preferred embodiment, therefore, the various flow rate signals Q can be generated at different time intervals with respect to each other, and in particular, such time intervals can increase progressively as the filter 4 is filled. In addition, such flow rate signals Q are advantageously of pulse type and all have the same intensity.

Otherwise, in accordance with an alternative embodiment, the flow rate measurement means 6 are configured to generate the flow rate signals Q at regular intervals over time, for example one per second. In such alternative embodiment, therefore, each flow rate signal Q can correspond to a different value of the volume of liquid to be tested L that passes through the measurement duct 3, and in particular the interval between two of the aforesaid consecutive volume values decreases as the filter 4 is filled. Therefore, in this case the flow rate signals Q are advantageously of analog type, and in particular are proportional to the flow rate measured by the flow rate measurement means 6. According to the idea underlying the present invention, the equipment 1 is also provided with a logic control unit 7 in data connection with the flow rate measurement means 6 in order to receive the plurality of flow rate signals Q.

The logic control unit 7 is also configured for generating one or more time values T on the basis of at least one of the aforesaid flow rate signals Q and for calculating a filtration parameter F of the liquid to be tested L on the basis of the time values T.

In particular, the filtration parameter F can be one or more from among the filterability index (IF), the modified filterability index (IFM), the maximum filterable volume (Vmax) and the Silt Density Index (SDI) and the logic control unit 7 is configured for calculating such filtration parameters F on the basis of formulas that are well-known and standardized in the field, and reported in the embodiments described hereinbelow.

The logic control unit 7 is provided with a memory module 70, in which the formulas are stored for calculating the filtration parameter F. In addition, the logic control unit 7 is provided with a user interface 71, through which an operator is susceptible of selecting the filtration parameter F to be calculated.

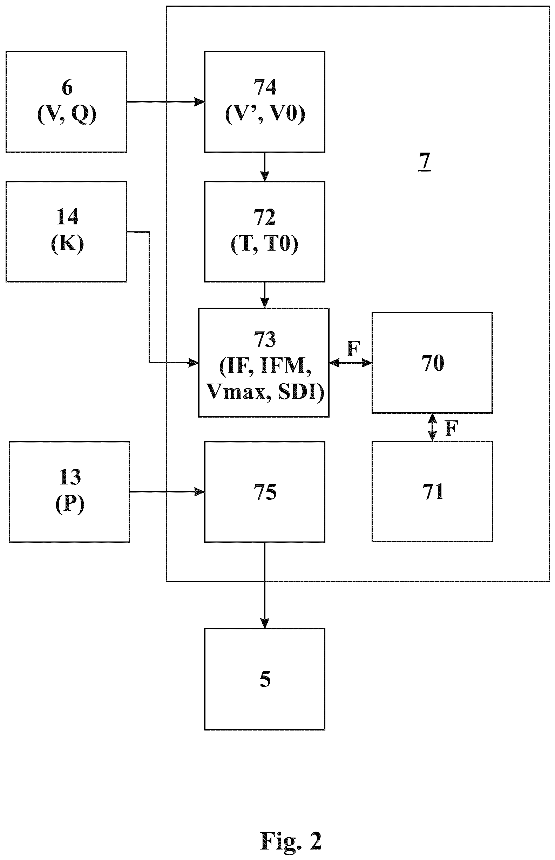

In accordance with the enclosed figure 2, the logic control unit 7 is advantageously also provided with a time measurement module 72 and with a calculation module 73. In particular, the time measurement module 72 is adapted to generate the time values T on the basis of at least one of the flow rate signals Q, and the calculation module 73 is adapted to calculate the filtration parameter F selected by the operator.

More in detail, each flow rate signal Q corresponds with the passage of a preset volume V of liquid to be tested L, the logic control unit 7 is configured for counting the number of the flow rate signals Q arriving from the flow rate measurement means 6 and for generating a time value T at the count of a preset number of flow rate signals Q. In particular, the time value T thus generated corresponds with the time necessary so that the passage of a reference volume V' takes place through the measurement duct 3, in which the reference volume V' is equal to the sum of the number of the preset volumes V that have passed at each counted flow rate signal Q.

The logic control unit 7 is configured for selecting the value of such reference volume V', and consequently the number of flow rate signals Q to be counted, on the basis of the filtration parameter F to be calculated. The logic control unit 7 is therefore advantageously provided with a count module 74 configured for counting the flow rate signals Q up to the attainment of such reference volume V' and adapted to communicate, with the time measurement module 72, the attainment of the reference volume V' itself, in order to allow such time measurement module 72 to generate the time value T necessary for calculating the desired filtration parameter F.

Of course, such count operation can be repeated multiple times during a same filtration test, e.g. in order to calculate the times necessary for the passage of different reference volumes V', functional for the calculation of the desired filtration parameter F.

In accordance with the enclosed figure 1, the equipment 1 also comprises pressure measurement means 13 placed to intercept the measurement duct 3. More in detail, the pressure measurement means 13 are preferably interposed between the flow rate measurement means 6 and the filter 4.

Advantageously, the pressure measurement means 13 are adapted to detect the pressure of the liquid to be tested L which flows in the measurement duct 3 and to generate a plurality of pressure signals P, corresponding to the detected pressure measurements. For example, the pressure measurement means 13 are of the type with membrane, in which the membrane is susceptible of being deformed following the pressure within the measurement duct 3 and is adapted to transform such deformation into an electrical signal by means of a piezoresistance.

In addition, the logic control unit 7 is advantageously in data connection with the pressure measurement means 13, so as to receive the pressure signals P, and is operatively connected to the suction pump 5 in order to control the operation thereof over time on the basis of the received pressure signals P.

In particular, the logic control unit 7 is provided with a control module 75, susceptible of receiving the pressure signals P and configured for verifying that the pressure within the measurement duct 3 is contained within a preset range of pressures. In addition, the control module 75 is adapted to control the suction speed of the suction pump 5 in a manner such to control the pressure within the measurement duct 3.

More in detail, the logic control unit 7 is advantageously adapted to control the operation over time of the suction pump 5, so as to maintain the pressure of the liquid to be tested L constant within the measurement duct 3, or at least maintain such pressure within the pre-established range of pressures.

Advantageously, the logic control unit 7 is also susceptible of sending to the suction pump 5 a turn-on signal, at the start of the filtration test, and a turn-off signal, at the end of the filtration test itself. In other words, therefore, the logic control unit 7 is programmed for controlling the entire operation of the filtration test executed with the equipment 1, object of the present invention.

For such purpose, the logic control unit 7 advantageously comprises at least one electronic control unit (e.g. a PLC) programmed for controlling the operation of the equipment 1 in an automated manner.

Advantageously, moreover, the logic control unit 7 is electrically connected to an external power supply (not illustrated in the enclosed figure) so as to execute the aforesaid operations. Preferably, moreover, the aforesaid external power supply is also electrically connected to the suction pump 5 and to the various measurement means arranged in the equipment 1.

Advantageously, the equipment 1 also comprises a battery backup and a recharging circuit, in a manner such to allow the operation of the equipment 1 even in the absence of the external power supply.

Advantageously, the equipment 1 also comprises temperature measurement means 14 placed to intercept the measurement duct 3. More in detail, the temperature measurement means 14 are preferably interposed between the flow rate measurement means 6 and the filter 4, and still more preferably they are interposed between the flow rate measurement means 6 and the pressure measurement means 13.

Otherwise, the temperature measurement means 14 can coincide with the flow rate measurement means 6, for example in the case of flow rate measurement means 6 of thermal dispersion type.

The temperature measurement means 14 are also adapted to detect the temperature of the liquid to be tested L which flows within the measurement duct 3 and to generate at least one temperature signal K corresponding to the detected temperature. Advantageously, moreover, the logic control unit 7 is in data connection with the temperature measurement means 14 in order to receive the temperature signal K and is configured for calculating the filtration parameter F of the liquid to be tested L on the basis of the time values T and of the temperature signal K.

More in detail, the logic control unit 7 is advantageously configured for correcting the filtration parameter F, calculated starting from the time values T, on the basis of the temperature signal K, since the filling capacity of the liquid to be tested L can vary with the variation of the temperature.

Otherwise, the logic control unit 7 can be configured in order to calculate the filtration parameter F by ignoring the temperature signal K, and in order to associate each filtration parameter F with the corresponding temperature value, so as to allow an operator to keep the test conditions under consideration.

In the latter case, the logic control unit 7 is advantageously configured for recording the filtration parameter F, calculated starting from the time values T, in combination with the temperature signal K. In particular, the logic control unit 7 is configured for compiling a record of the filtration parameters F calculated and of the temperature measurements associated with the temperature signals K.

In accordance with the enclosed figure 1, the equipment 1 is advantageously provided with a safety duct 15, hydraulically connected to the measurement duct 3 and adapted to protect the suction pump 5. More in detail, the safety duct 15 is extended between a first end 16 and a second end 17, in which the first end 16 is hydraulically connected to the delivery section 52 of the suction pump 5 and the second end 17 is hydraulically connected to the suction section 51 of the suction pump 5 itself.

Preferably, moreover, as illustrated in figure 1, the first end 16 of the safety duct 15 is in fluid connection with the measurement duct 3 upstream of the flow rate measurement means 6, and the second end 17 is in fluid connection with the suction duct 10 upstream of the suction pump 5.

Advantageously, the equipment 1 is also provided with a safety valve 18, preferably of relief type, which is placed to intercept the safety duct 15 and is adapted to allow the passage of the liquid to be tested L within the safety duct 15 itself at the attainment of a preset threshold pressure P' of the liquid to be tested L within the measurement duct 3. More in detail, the safety valve 18 is advantageously provided with a shutter (not illustrated in the enclosed figure) susceptible of being moved by the pressure present within the measurement duct 3. In particular, upon attaining the threshold pressure P', such shutter is susceptible of passing from a blocking configuration, in which it obstructs the first end 16 of the safety duct 15, to a discharge configuration, in which it frees the aforesaid first end 16 and allows the liquid to be tested L to flow at its interior. In this manner, the safety valve 18 allows creating a recirculation of the liquid to be tested L, discharging the latter upstream of the suction pump 5 if an overly high pressure is reached downstream of the same. In other words, therefore, the safety valve 18 and the safety duct 15 are adapted to prevent the suction pump 5 from continuing to convey liquid to be tested L within the measurement duct 3 if the filter 4 is filled.

Advantageously, moreover, once the pressure within the measurement duct 3 newly falls below the threshold pressure P', the shutter of the safety valve 18 is susceptible of returning from the discharge configuration to the blocking configuration, in a manner such that the liquid to be tested L once again returns to flowing only within the measurement duct 3.

Advantageously, moreover, the logic control unit 7 is also programmed for communicating the calculated filtration parameters F, for example through the user interface 71, with the operator who is using the equipment 1.

Also forming the object of the present invention is a process for executing filtration tests on a liquid to be tested L, in particular by means of the equipment 1, object of the present invention, regarding which the same reference numbers will be maintained for the sake of description simplicity.

In particular, the present process provides for arranging the above-described equipment 1. More in detail, according to the invention, the present process provides for a first step of arranging a measurement duct 3, extended between an inlet section 31 and an outlet section 32, and preferably also a first container 2 containing a liquid to be tested L. In addition, in such first arranging step the inlet section 31 of the measurement duct 3 is hydraulically connected to the first container 2.

In addition, the present process provides for a second step of arranging a filter 4, which is hydraulically connected to the measurement duct 3, preferably at the outlet section 32 thereof.

For example, the aforesaid second arranging step advantageously provides for selecting a specific filter 4 to be tested and for inserting such filter 4 within a containment body 9 of the above-described type, which is thus associated with the measurement duct 3, for example via screwing.

In accordance with the idea underlying the present invention, the present process also provides for a third step of arranging a suction pump 5, which is hydraulically connected to the inlet section 31 of the measurement duct 3 and preferably also to the first container 2, for example by means of the arrangement of a suction duct 10.

According to the invention, the process also comprises a fourth step of arranging flow rate measurement means 6, preferably of the above-described type, which are placed to intercept the measurement duct 3.

Preferably, moreover, the third step of arranging the suction pump 5 is executed before connecting the measurement duct 3 to the first container 2 in the first arranging step. The aforesaid arranging steps, therefore, are numbered for the sake of description simplicity, and the order with which they are numbered does not necessarily coincide with the order of execution thereof.

Advantageously, moreover, the present process can comprise further arranging steps, aimed to associate the various elements that constitute the above-described equipment 1 with each other. For example, in order to hydraulically connect the pressure measurement means 13 and the temperature measurement means 14 to the measurement duct 3.

Following the various arranging steps, the present method provides for a test step, of the type described hereinbelow.

More in detail, according to the idea underlying the present invention, the present process provides for a suctioning step, in which the suction pump 5 suctions the liquid to be tested L from the first container 2, in particular through the suction duct 10, and conveys it into the measurement duct 3.

The present process also provides for a step of filtering the liquid to be tested L by means of the filter 4. In particular, a permeate fraction of the liquid to be tested L itself that traverses the filter 4 is discharged into a second container 8, advantageously arranged beyond the outlet section 32 of the measurement duct 3.

Preferably, the aforesaid steps of suctioning and filtering occur continuously up to the end of the aforesaid test step.

According to the invention, the process also provides for a detection step, in which the flow rate measurement means 6 detect the flow rate of the liquid to be tested L which flows in the measurement duct 3. Preferably, the aforesaid detection step also occurs continuously up to the end of the test step, and in particular it substantially occurs simultaneously with the suction and filtering steps.

In the aforesaid measurement step, moreover, the flow rate measurement means 6 generate a plurality of flow rate signals Q, corresponding to the detected measurements, and send such flow rate signals Q to a logic control unit 7, in particular of the above-described type.

The present process also provides for a processing step, in which the logic control unit 7 generates one or more time values T on the basis of the flow rate signals Q and calculates a filtration parameter F of the liquid to be tested L on the basis of such time values T.

Advantageously, in accordance with the preferred embodiment described above, in which each flow rate signal Q generated by the flow rate measurement means 6 corresponds with the passage of a preset volume V of liquid to be tested L, during the processing step the logic control unit 7 counts the number of flow rate signals Q arriving from the flow rate measurement means 6, generates the time value T at the count of a preset number of such flow rate signals Q and calculates the filtration parameter F on the basis of the time values T.

In this manner, in such preferred embodiment, the logic control unit 7 continues to count the flow rate signals Q up to attaining a specific reference volume V' (equal to the sum of the number of the preset volumes V that have passed at each counted flow rate signal Q) and calculates the filtration parameter F on the basis of such reference volume V'. Advantageously, in this manner, the logic control unit 7 calculates the filtration parameter F on the basis of the exact time value T corresponding with the passage of the reference volume V'.

In accordance with the alternative embodiment described above, in which each flow rate signal Q is generated at regular time intervals, during the processing step the logic control unit 7 interpolates the flow rate values measured by the flow rate measurement means 6 so as to obtain the time value T corresponding to the reference volume V' necessary for calculating the desired filtration parameter F. In such alternative embodiment, therefore, the logic control unit 7 calculates the filtration parameter F on the basis of an approximate time value T and therefore also the filtration parameter F is approximate.

Advantageously, moreover, the present process provides for repeating, continuously over time, the detection step so as to detect multiple flow rate signals Q corresponding to multiple time values T, necessary for calculating the desired filtration parameter F. More in detail, the logic control unit 7 advantageously generates a time value T for each corresponding reference volume V' necessary for calculating the filtration parameter F selected by the operator.

For example, if an operator selects calculating the filterability index (IF), the logic control unit 7 generates the time values T corresponding to reference volumes V' equal to 200 ml and 400 ml. Otherwise, if the operator selects calculating the modified filterability index (IFM), the logic control unit 7 also generates the time value T corresponding to the reference volume V' equal to 600 ml.

Advantageously, moreover, the present process additionally provides for a starting step, preceding the processing step, in which the logic control unit 7 counts a preset initial number of flow rate signals Q and generates an initial time value T0 from which the processing step starts.

More in detail, in such starting step the logic control unit 7 counts the number of flow rate signals Q necessary for attaining an initial volume V0 for starting the equipment 1, and subsequently upon attaining such initial volume V0 starts the count of the flow rate signals Q necessary for obtaining the reference volume V'.

In particular, the aforesaid initial volume V0 is the volume of liquid to be tested L necessary for bringing the equipment 1 to standard operating conditions, i.e. necessary for wetting the filter 4, and for reaching a minimum operating pressure within the measurement duct 3.

In accordance with a first embodiment for calculating the filterability index (IF), the method provides for arranging a filter 4 with diameter equal to 25 mm and micrometry comprised between 0.45 and 0.65 $\mu$m. In addition, the method, as anticipated above, provides for generating a first time T1 at the passage of a first reference volume V' 1 equal to 200 ml starting from the initial time T0. By way of example, with preset volume V equal to 1 ml, the first time T1 is generated by the logic control unit 7 after the latter has counted a number equal to 200 flow rate signals Q, corresponding to the consecutive passage of respective preset volumes V at the flow rate measurement means 6.

In addition, the method, as anticipated above, provides for generating a second time T2 at the passage of a second reference volume V'2 equal to 400 ml starting from the initial time T0. By way of example, with preset volume V equal to 1 ml, the second time T2 is generated by the logic control unit 7 after the latter has counted a number equal to 400 flow rate signals Q, corresponding to the consecutive passage of respective preset volumes V at the flow rate measurement means 6.

The filterability index (IF) is then calculated with the formula 1, reported hereinbelow:

$$IF = T2 - 2 \cdot T1 \qquad\qquad\qquad \text{(formula 1)}$$

In accordance with a second embodiment for calculating the modified filterability index (IFM), the method, as anticipated above, provides for generating, in addition to the times T1 and T2 described in the first example, also a third time T3 at the passage of a third reference volume V'3 equal to 600 ml starting from the initial time T0. By way of example, with preset volume V equal to 1 ml, the third time T3 is generated by the logic control unit 7 after the latter has counted a number equal to 600 flow rate signals Q, corresponding to the consecutive passage of respective preset volumes V at the flow rate measurement means 6.

The modified filterability index (IFM) is then calculated with the formula 2, reported hereinbelow:

$$IFM = (T3 - T1) - 2 \cdot (T2 - T1) \qquad \text{(formula 2)}$$

In accordance with a third embodiment for calculating the maximum filterable volume (Vmax), the method provides for arranging a filter 4 with diameter equal to 47 mm of the same material used in the cartridge filters that must be employed in the filtration process. In addition, the method provides for generating a plurality of times Ti, Ti+1, ..., Tn at the passage of a plurality of reference volumes V'i, V'i+1, ..., V'n starting from the initial time T0.

Subsequently, the logic control unit 7 calculates the corresponding values Ti/V'i and 1/Ti for each reference volume and each generated time and interpolates such values on a graph with 1/Ti on the x-axis and Ti/V'i on the y-axis in order to obtain an interpolation line of equation indicated in formula 3, reported hereinbelow:

$$Ti/V'i = A \cdot Ti + B \qquad \text{(formula 3)}$$

in which A and B are coefficients obtained by means of the interpolation and in particular A is the angular coefficient of the line.

The maximum filterable volume (Vmax) is then calculated by the logic control unit 7 with the formula 4, reported hereinbelow:

$$Vmax = 1/A \qquad \text{(formula 4)}$$

Alternatively, the maximum filterable volume (Vmax) can be calculated by the logic control unit 7 on the basis of the data obtained with the first embodiment by means of formula 5, reported hereinbelow:

$$Vmax = 400 + 400 \cdot T1/IF \qquad \text{(formula 5)}$$

Of course, further embodiment variants are possible according to which the logic control unit can also calculate the liquid flow rates on the basis of the times Ti, Ti+1, ..., Tn generated and the reference volumes V'i, V'i+1, ..., V'n and use such values for calculating the maximum filterable volume or other filtration parameters.

In all of the above-described examples, the pressure is maintained constant by the logic control unit 7 by means of the pressure measurements means 13.

In accordance with a fourth embodiment for calculating the Silt Density Index (SDI), in particular for the filtration of water, the method provides for arranging a filter 4 with diameter equal to 47 mm and with micrometry equal to 0.45 $\mu$m. In addition, the method provides for starting the count of the flow rate signals Q after a time interval from the start of the equipment 1 (and thus from the start of the flow of liquid to be tested L) generally equal to 15 minutes. Hence, in this case the time T0 is generated as a function of the aforesaid time interval rather than on the basis of a preset initial number of flow rate signals Q. Subsequently, after the start of the count, the logic control unit generates an initial time value Ta at the passage of an initial reference volume V'a equal to 0.5 liters starting from the initial time T0.

Subsequently, the method provides for waiting a reference time interval Ttot, generally equal to 15 minutes, starting from the generation of Ta and for starting a second count of the flow rate signals Q subsequent to such reference time interval Ttot. Subsequently, after the start of the second count, the logic control unit generates a final time value Tb at the passage of a final reference volume V'b equal to 0.5 liters starting from the start of the second count.

The Silt Density Index (SDI) is then calculated by the logic control unit 7 with the formula 6, reported hereinbelow:

$$SDI = [(1 - Ta)/Tb] \cdot (100/Ttot) \qquad \text{(formula 6)}$$

in which Ta, Tb and Ttot are expressed in minutes.

In the above-described fourth example, the pressure is maintained constant at a value of 2 bar by the logic control unit 7, by means of the pressure measurements means 13. The invention thus conceived therefore attains the pre-established objects.

**Claims**

1. Equipment for executing filtration tests on a liquid to be tested (L), which is susceptible of being hydraulically connected to a first container (2) containing the liquid to be tested (L) and comprises:

   - a measurement duct (3), which is extended between an inlet section (31), placed in fluid communication with the first container (2), and an outlet section (32), and in which the liquid to be tested (L) is susceptible of flowing;
   - a filter (4) hydraulically connected to said measurement duct (3) and adapted to filter the liquid to be tested (L);
   - a suction pump (5), which is hydraulically connected to the inlet section (31) of said measurement duct (3) and is adapted to suction the liquid to be tested (L) from the first container (2) and to convey it into said measurement duct (3);
   - flow rate measurement means (6), which are placed to intercept said measurement duct (3) and are adapted to detect the flow rate of the liquid to be tested (L) which flows in said measurement duct (3) and to generate a corresponding plurality of flow rate signals (Q);
   - a logic control unit (7), which is in data connection with said flow rate measurement means (6) in order to receive said plurality of flow rate signals (Q) and is configured for:

      - generating one or more time values (T) on the basis of at least one of said flow rate signals (Q);
      - calculating a filtration parameter (F) of the liquid to be tested (L) on the basis of said one or more time values (T);

   wherein each of said flow rate signals (Q) corresponds with the passage of a preset volume (V) of liquid to be tested (L) through said measurement duct (3); wherein said logic control unit (7) is configured for counting the number of said flow rate signals (Q) arriving from said flow rate measurement means (6) and for generating one of said time values (T) at the count of a preset number of said flow rate signals (Q);
   wherein said time value (T) thus generated corresponds with the time necessary so that the passage of a reference volume (V') takes place through said measurement duct (3), wherein said reference volume (V') is equal to the sum of the number of said preset volumes (V) that have passed at each counted flow rate signal (Q);
   wherein said logic control unit (7) is configured for selecting the value of said reference volume (V'), and consequently the number of said flow rate signals (Q) to be counted, on the basis of said filtration parameter (F) to be calculated;
   said equipment (1) being **characterized in that** said logic control unit (7) is provided with:

      - a memory module (70), in which the formulas are stored for calculating the filtration parameter (F); said filtration parameter (F) being one or more from among: filterability index (IF), modified filterability index (IFM), maximum filterable volume (Vmax) and Silt Density Index (SDI);
      - a user interface (71), through which an operator is susceptible of selecting the filtration parameter (F) to be calculated.

2. Equipment according to claim 1, **characterized in that** it comprises pressure measurement means (13), which are placed to intercept said measurement duct (3), are adapted to detect the pressure of the liquid to be tested (L) which flows in said measurement duct (3) and to generate a corresponding plurality of pressure signals (P).

3. Equipment according to claim 2, **characterized in that** said logic control unit (7) is in data connection with said pressure measurement means (13) in order to receive said plurality of pressure signals (P), and is operatively connected to said suction pump (5) in order to maintain the pressure of the liquid to be tested (L) constant over time within said measurement duct (3).

4. Equipment according to one of the preceding claims, **characterized in that** it comprises temperature measurement means (14), which are placed to intercept said measurement duct (3), are adapted to detect the temperature of the liquid to be tested (L) which flows in said measurement duct (3) and to generate at least one corresponding temperature signal (K);
   said logic control unit (7) being in data connection with said temperature measurement means (14) in order to receive said at least one temperature signal (K) and being configured for recording values of said temperature signals (K) associated with corresponding said filtration parameters (F).

5. Equipment according to any one of the preceding claims, **characterized in that** it comprises:

- a safety duct (15), hydraulically connected to said measurement duct (3) and extended between:

- a first end (16), hydraulically connected to a delivery section of said suction pump (5), and
- a second end (17), hydraulically connected to a suction section (51) of said suction pump (5);

- a safety valve (18), placed to intercept said safety duct (15) and adapted to allow the passage of the liquid to be tested (L) in said safety duct (15) at the attainment of a preset threshold pressure (P') of the liquid to be tested (L) within said measurement duct (3).

6. Process for executing filtration tests on a liquid to be tested (L), which provides for:

- a first step of arranging a measurement duct (3) extended between an inlet section (31) and an outlet section (32), in which said inlet section (31) is hydraulically connected to a first container (2) containing a liquid to be tested (L);
- a second step of arranging a filter (4), in which said filter (4) is hydraulically connected to said measurement duct (3);
- a third step of arranging a suction pump (5), in which said suction pump (5) is hydraulically connected to the inlet section (31) of said measurement duct (3);
- a fourth step of arranging flow rate measurement means (6), in which said flow rate measurement means (6) are placed to intercept said measurement duct (3);
- a suctioning step, in which said suction pump (5) suctions the liquid to be tested (L) from the first container (2) and conveys it into said measurement duct (3);
- a step of filtering the liquid to be tested (L) by means of said filter (4);
- a detection step, in which said flow rate measurement means (6) detect the flow rate of the liquid to be tested (L) which flows in said measurement duct (3), generate a plurality of corresponding flow rate signals (Q) and send said flow rate signals (Q) to a logic control unit (7);
- a processing step, in which said logic control unit (7) generates one or more time values (T) on the basis of said flow rate signals (Q) and calculates a filtration parameter (F) of the liquid to be tested (L) on the basis of said one or more time values (T);
wherein, in said detection step, said flow rate measurement means (6) generate each of said flow rate signals (Q) at the passage of a preset volume (V);
in said processing step, said logic control unit (7):

- counts the number of said flow rate signals (Q) arriving from said flow rate measurement means (6),
- generates one of said one or more time values (T) at the count of a preset number of said flow rate signals (Q);
- calculates said filtration parameter (F) on the basis of said one or more time values (T);

wherein said logic control unit (7) continues to count the flow rate signals (Q) up to attaining a specific reference volume (V') equal to the sum of the number of the preset volumes (V) that have passed at each counted flow rate signal (Q) and calculates said filtration parameter (F) on the basis of said time value (T) corresponding with the passage of said reference volume (V');
wherein said logic control unit (7) selects the value of said reference volume (V'), and consequently the number of said flow rate signals (Q) to be counted, on the basis of said filtration parameter (F) to be calculated;
said process being **characterized in that** said logic control unit (7) is provided with:

- a memory module (70), in which the formulas are stored for calculating the filtration parameter (F); said filtration parameter (F) being one or more from among: filterability index (IF), modified filterability index (IFM), maximum filterable volume (Vmax) and Silt Density Index (SDI);
- a user interface (71), through which an operator is susceptible of selecting the filtration parameter (F) to be calculated.

7. Process according to claim 8, **characterized in that** it provides for a starting step, before said processing step, in which said logic control unit (7) counts a preset initial number of said flow rate signals (Q) and generates an initial time value (T0) from which said processing step starts.

**Patentansprüche**

1. Vorrichtung zur Durchführung von Filtrationstests an einer zu testenden Flüssigkeit (L), die geeignet ist, hydraulisch an einen ersten Behälter (2) angeschlossen zu werden, der die zu testende Flüssigkeit (L) enthält und Folgendes umfasst:

   - einen Messkanal (3), der zwischen einem Eintrittsabschnitt (31), der mit dem ersten Behälter (2) in Fluidverbindung steht, und einem Austrittsabschnitt (32) verläuft, und in dem die zu testende Flüssigkeit (L) zu fließen geeignet ist;
   - einen Filter (4), der hydraulisch an den genannten Messkanal (3) angeschlossen und geeignet ist, die zu testende Flüssigkeit (L) zu filtern;
   - eine Saugpumpe (5), die hydraulisch an den Eintrittsabschnitt (31) des genannten Messkanals (3) angeschlossen und geeignet ist, die zu testende Flüssigkeit (L) aus dem ersten Behälter (2) anzusaugen und sie in den genannten Messkanal (3) zu leiten;
   - Durchflussmesselemente (6), die den genannten Messkanals (3) erfassend angeordnet und geeignet sind, den Durchfluss der zu testenden Flüssigkeit (L) zu messen, die in dem genannten Messkanal (3) fließt, und eine entsprechende Vielzahl von Durchflusssignalen (Q) zu erzeugen;
   - eine logische Steuerung (7), die in Datenverbindung mit den genannten Durchflussmesselementen (6) steht, um die genannte Vielzahl von Durchflusssignalen (Q) zu empfangen, und darauf ausgelegt ist:

      - einen oder mehrere Zeitwerte (T) auf Grundlage mindestens eines der genannten Durchflusssignale (Q) zu erzeugen;
      - einen Filtrationsparameter (F) der zu testenden Flüssigkeit (L) auf Grundlage der genannten einen oder mehreren Zeitwerte (T) zu berechnen;

   wobei jedes der genannten Durchflusssignale (Q) dem Durchsatz eines voreingestellten Volumens (V) zu testender Flüssigkeit (L) über den genannten Messkanal (3) entspricht; wobei die genannte logische Steuerung (7) darauf ausgelegt ist, die Anzahl der von den genannten Durchflussmesselementen (6) kommenden genannten Durchflusssignale (Q) zu zählen und einen der genannten Zeitwerte (T) bei der Zählung einer voreingestellten Anzahl der genannten Durchflusssignale (Q) zu erzeugen;
   wobei der so erzeugte genannte Zeitwert (T) der Zeit entspricht, die erforderlich ist, damit der Durchsatz eines Bezugsvolumens (V') über den genannten Messkanal (3) erfolgt, wobei das Bezugsvolumen (V') der Summe der Anzahl der genannten voreingestellten Volumen (V) entspricht, die bei jedem gezählten Durchflusssignal (Q) durchgesetzt wurden;
   wobei die genannte logische Steuerung (7) darauf ausgelegt ist, den Wert des genannten Bezugsvolumens (V'), und folglich die Anzahl der zu zählenden genannten Durchflusssignale (Q), auf Grundlage des zu berechnenden genannten Filtrationsparameters (F) zu wählen;
   wobei die genannte Vorrichtung (1) **dadurch gekennzeichnet ist, dass** die genannte logische Steuerung (7) mit Folgendem ausgestattet ist:

      - einem Speichermodul (70), in dem die Formeln zur Berechnung des Filtrationsparameters (F) gespeichert sind; wobei der genannte Filtrationsparameter (F) einen oder mehrere unter folgenden darstellt: Filtrierbarkeitsindex (IF), modifizierter Filtrierbarkeitsindex (IFM), maximal filtrierbares Volumen (Vmax) und Silt Density Index (SDI);
      - einer Benutzerschnittstelle (71), über die ein Bediener in der Lage ist, den zu berechnenden Filtrationsparameter (F) zu wählen.

2. Vorrichtung nach Anspruch 1, **dadurch gekennzeichnet, dass** sie Druckmesselemente (13) umfasst, die den genannten Messkanal (3) erfassend angeordnet sind, geeignet sind, den Druck der zu testenden Flüssigkeit (L) zu messen, die in dem genannten Messkanal (3) fließt, und eine entsprechende Vielzahl von Drucksignalen (P) zu erzeugen.

3. Vorrichtung nach Anspruch 2, **dadurch gekennzeichnet, dass** die genannte logische Steuerung (7) in Datenverbindung mit den Druckmesselementen (13) steht, um die genannte Vielzahl an Drucksignalen (P) zu empfangen, und operativ an die genannte Saugpumpe (5) angeschlossen ist, um den Druck der zu testenden Flüssigkeit (L) im Inneren des genannten Messkanals (3) im Zeitablauf konstant zu halten.

4. Vorrichtung nach einem der vorangegangenen Ansprüchen, **dadurch gekennzeichnet, dass** sie Temperaturmesselemente (14) umfasst, die den genannten Messkanal (3) erfassend angeordnet sind, geeignet sind, die Temperatur der zu testenden Flüssigkeit (L) zu messen, die in dem genannten Messkanal (3) fließt, und mindestens ein entsprechendes Temperatursignal (K) zu erzeugen;

wobei die genannte logische Steuerung (7) in Datenverbindung mit den genannten Temperaturmesselementen (14) steht, um mindestens ein Temperatursignal (K) zu empfangen, und darauf ausgelegt ist, Werte der genannten Temperatursignale (K) zu registrieren, die mit entsprechenden genannten Filtrationsparametern (F) verknüpft sind.

5. Vorrichtung nach einem beliebigen der vorangegangenen Ansprüche, **dadurch gekennzeichnet, dass** sie Folgendes umfasst:

- einen Sicherheitskanal (15), der hydraulisch an den genannten Messkanal (3) angeschlossen ist und zwischen Folgendem verläuft:

- einem ersten Ende (16), das hydraulisch an einen Zuleitungsabschnitt der genannten Saugpumpe (5) angeschlossen ist, und
- einem zweiten Ende (17), das hydraulisch an einen Saugabschnitt (51) der genannten Saugpumpe (5) angeschlossen ist;

- einem Sicherheitsventil (18), das den genannten Sicherheitskanal (15) erfassend angeordnet und geeignet ist, den Durchsatz der zu testenden Flüssigkeit (L) in dem genannten Sicherheitskanal (15) beim Erreichen eines voreingestellten Schwellendrucks (P') der zu testenden Flüssigkeit (L) im Inneren des genannten Messkanals (3) zu gestatten.

6. Verfahren zur Durchführung von Filtrationstests an einer zu testenden Flüssigkeit (L), das Folgendes vorsieht:

- einen ersten Schritt der Vorbereitung eines Messkanals (3), der zwischen einem Eintrittsabschnitt (31) und einem Austrittsabschnitt (32) verläuft, wobei der genannte Eintrittsabschnitt (31) hydraulisch an einen die zu testende Flüssigkeit (L) enthaltenden ersten Behälter (2) angeschlossen ist;
- einen zweiten Schritt der Vorbereitung eines Filters (4), wobei der genannte Filter (4) hydraulisch an den genannten Messkanal (3) angeschlossen ist;
- einen dritten Schritt der Vorbereitung einer Saugpumpe (5), wobei die genannte Saugpumpe (5) hydraulisch an den Eintrittsabschnitt (31) des genannten Messkanals (3) angeschlossen ist;
- einen vierten Schritt der Vorbereitung von Durchflussmesselementen (6), wobei die genannten Durchflussmesselemente (6) den genannten Messkanal (3) erfassend angeordnet sind;
- einen Saugschritt, bei dem die genannte Saugpumpe (5) die zu testende Flüssigkeit (L) aus dem ersten Behälter (2) ansaugt und ihn in den genannten Messkanal (3) leitet;
- einen Schritt des Filterns der zu testenden Flüssigkeit (L) mittels des genannten Filters (4);
- einen Messschritt, bei dem die genannten Durchflussmesselemente (6) den Durchfluss der zu testenden Flüssigkeit (L) messen, die in dem genannten Messkanal (3) fließt, eine Vielzahl von entsprechenden Durchflusssignalen (Q) erzeugen und die genannten Durchflusssignale (Q) an eine logische Steuerung (7) übertragen;
- einen Verarbeitungsschritt, bei dem die genannte logische Steuerung (7) einen oder mehrere Zeitwerte (T) auf Grundlage der genannten Durchflusssignale (Q) erzeugt und einen Filtrationsparameter (F) der zu testenden Flüssigkeit (L) auf Grundlage der genannten einen oder mehreren Zeitwerte (T) berechnet;

wobei bei dem genannten Messschritt die genannten Durchflussmesselemente (6) jeweils die genannten Durchflusssignale (Q) beim Durchsatz eines voreingestellten Volumens (V) erzeugen;

wobei bei dem genannten Verarbeitungsschritt die genannte logische Steuerung (7):

- die Anzahl der von den genannten Durchflussmesselementen (6) kommenden genannten Durchflusssignale (Q) zählt,
- einen der genannten einen oder mehreren Zeitwerte (T) bei der Zählung einer voreingestellten Anzahl der genannten Durchflusssignale (Q) erzeugt;
- den genannten Filtrationsparameter (F) auf Grundlage der genannten einen oder mehreren Zeitwerte (T) berechnet;

wobei die genannte logische Steuerung (7) die Durchflusssignale (Q) bis zum Erreichen eines bestimmten Bezugsvolumens (V') in Höhe der Summe der Anzahl der voreingestellten Volumen (V) weiter zählt, die bei jedem gezählten Durchflusssignal (Q) durchgesetzt wurden, und den genannten Filtrationsparameter (F) auf Grundlage des genannten Zeitwerts (T) berechnet, der dem Durchsatz des genannten Referenzvolumens (V') entspricht;

wobei die genannte logische Steuerung (7) den Wert des genannten Bezugsvolumens (V'), und folglich die Anzahl der zu zählenden genannten Durchflusssignale (Q), auf Grundlage des zu berechnenden genannten Filtrationsparameters (F) wählt;

wobei das genannte Verfahren **dadurch gekennzeichnet ist, dass** die genannte logische Steuerung (7) mit Folgendem ausgestattet ist:

- einem Speichermodul (70), in dem die Formeln zur Berechnung des Filtrationsparameters (F) gespeichert sind; wobei der genannte Filtrationsparameter (F) einen oder mehrere unter folgenden darstellt: Filtrierbarkeitsindex (IF), modifizierter Filtrierbarkeitsindex (IFM), maximal filtrierbares Volumen (Vmax) und Silt Density Index (SDI);
- einer Benutzerschnittstelle (71), über die ein Bediener in der Lage ist, den zu berechnenden Filtrationsparameter (F) zu wählen.

7. Verfahren nach Anspruch 8, **dadurch gekennzeichnet, dass** es einen der genannten Verarbeitungsphase vorausgehenden Startschritt vorsieht, bei dem die genannte logische Steuerung (7) eine voreingestellte anfängliche Anzahl der genannten Durchflusssignale (Q) zählt und einen anfänglichen Zeitwert (T0) erzeugt, ab dem der genannte Verarbeitungsschritt beginnt.

## Revendications

1. Équipement d'exécution de tests de filtration sur un liquide à tester (L), qui est susceptible d'être relié hydrauliquement à un premier récipient (2) contenant le liquide à tester (L) et comprenant :

- un conduit de mesure (3) qui s'étend entre une section d'entrée (31), placée en communication fluidique avec le premier récipient (2), et une section de sortie (32), et dans lequel le liquide à tester (L) est susceptible de s'écouler ;
- un filtre (4) relié hydrauliquement audit conduit de mesure (3) et apte à filtrer le liquide à tester (L) ;
- une pompe d'aspiration (5) qui est reliée hydrauliquement à la section d'entrée (31) dudit conduit de mesure (3) et est apte à aspirer le liquide à tester (L) à partir du premier récipient (2) et à le transporter dans ledit conduit de mesure (3) ;
- des moyens de mesure de débit (6), qui sont placés pour intercepter ledit conduit de mesure (3) et sont aptes à détecter le débit du liquide à tester (L) qui s'écoule dans ledit conduit de mesure (3) et à générer une pluralité correspondante de signaux de débit (Q) ;
- une unité de commande logique (7), qui est en connexion de données avec lesdits moyens de mesure de débit (6) pour recevoir ladite pluralité de signaux de débit (Q) et est configurée pour :

- générer une ou plusieurs valeurs de temps (T) sur la base d'au moins un desdits signaux de débit (Q) ;
- calculer un paramètre de filtration (F) du liquide à tester (L) sur la base desdites une ou plusieurs valeurs de temps (T) ;

dans lequel chacun desdits signaux de débit (Q) correspond au passage d'un volume par défaut (V) de liquide à tester (L) à travers ledit conduit de mesure (3) ; dans lequel ladite unité de commande logique (7) est configurée pour prendre en compte le nombre desdits signaux de débit (Q) arrivant desdits moyens de mesure de débit (6) et pour générer l'une desdites valeurs de temps (T) en correspondance avec la prise en compte d'un nombre par défaut desdits signaux de débit (Q) ;

dans lequel ladite valeur de temps (T) ainsi générée correspond au temps nécessaire pour que le passage d'un volume de référence (V') à travers ledit conduit de mesure (3) se produise, dans lequel ledit volume de référence (V') est égal à la somme du nombre desdits volumes par défaut (V) qui sont passés en correspondance avec chaque signal de débit (Q) pris en compte ;

dans lequel ladite unité de commande logique (7) est configurée pour sélectionner la valeur dudit volume de référence (V'), et par conséquent le nombre desdits signaux de débit (Q) à prendre en compte, sur la base dudit paramètre de filtration (F) à calculer ; ledit équipement (1) étant **caractérisé en ce que** ladite unité de commande logique (7) est pourvue de :

- un module de mémoire (70), dans lequel les formules pour le calcul du paramètre de filtration (F) sont mémorisées ; ledit paramètre de filtration (F) étant l'un ou plusieurs parmi : l'indice de filtrabilité (IF), indice de filtrabilité modifié (IFM), volume maximum filtrable (Vmax) et indice de colmatage (Silt Density Index ou SDI) ;
- une interface utilisateur (71), à travers laquelle un opérateur est susceptible de sélectionner le paramètre de filtration (F) à calculer.

2. Équipement selon la revendication 1, **caractérisé en ce qu'**il comprend des moyens de mesure de pression (13), qui sont placés pour intercepter ledit conduit de mesure (3), sont aptes à détecter la pression du liquide à tester (L) qui s'écoule dans ledit conduit de mesure (3) et à générer une pluralité correspondante de signaux de pression (P).

3. Équipement selon la revendication 2, **caractérisé en ce que** ladite unité de commande logique (7) est en connexion de données avec lesdits moyens de mesure de pression (13) pour recevoir ladite pluralité de signaux de pression (P), et est reliée fonctionnellement à ladite pompe d'aspiration (5) pour maintenir la pression du liquide à tester (L) constante dans le temps à l'intérieur dudit conduit de mesure (3).

4. Équipement selon l'une des revendications précédentes, **caractérisé en ce qu'**il comprend des moyens de mesure de température (14), qui sont placés pour intercepter ledit conduit de mesure (3), sont aptes à détecter la température du liquide à tester (L) qui s'écoule dans ledit conduit de mesure (3) et à générer au moins un signal de température (K) correspondant :
ladite unité de commande logique (7) étant en connexion de données avec lesdits moyens de mesure de température (14) pour recevoir ledit au moins un signal de température (K) et étant configurée pour enregistrer des valeurs desdits signaux de température (K) associés auxdits paramètres de filtration (F) correspondants.

5. Équipement selon l'une quelconque des revendications précédentes, **caractérisé en ce qu'**il comprend :

- un conduit de sécurité (15), relié hydrauliquement audit conduit de mesure (3) et s'étendant entre :

  - une première extrémité (16), reliée hydrauliquement à une section de refoulement de ladite pompe d'aspiration (5), et
  - une seconde extrémité (17), reliée hydrauliquement à une section d'aspiration (51) de ladite pompe d'aspiration (5) ;

- une soupape de sûreté (18), placée pour intercepter ledit conduit de sécurité (15) et apte à permettre le passage du liquide à tester (L) dans ledit conduit de sécurité (15) en correspondance avec l'obtention d'une pression de seuil (P') par défaut du liquide à tester (L) à l'intérieur dudit conduit de mesure (3).

6. Procédé d'exécution de tests de filtration sur un liquide à tester (L), qui prévoit :

- une première étape de prédisposition d'un conduit de mesure (3) se développant entre une section d'entrée (31) et une section de sortie (32), dans lequel ladite section d'entrée (31) est reliée hydrauliquement à un premier récipient (2) contenant un liquide à tester (L) ;
- une seconde étape de prédisposition d'un filtre (4), dans laquelle ledit filtre (4) est relié hydrauliquement audit conduit de mesure (3) ;
- une troisième étape de prédisposition d'une pompe d'aspiration (5), dans laquelle ladite pompe d'aspiration (5) est reliée hydrauliquement à la section d'entrée (31) dudit conduit de mesure (3) ;
- une quatrième étape de prédisposition des moyens de mesure de débit (6), dans laquelle lesdits moyens de mesure de débit (6) sont placés pour intercepter ledit conduit de mesure (3) ;
- une étape d'aspiration, dans laquelle ladite pompe d'aspiration (5) aspire le liquide à tester (L) à partir du premier récipient (2) et le transporte dans ledit conduit de mesure (3) ;
- une étape de filtration du liquide à tester (L) par le biais dudit filtre (4) ;
- une étape de détection, dans laquelle lesdits moyens de mesure de débit (6) détectent le débit du liquide à tester (L) qui s'écoule dans ledit conduit de mesure (3), génèrent une pluralité de signaux de débit (Q) correspondants et envoient lesdits signaux de débit (Q) à une unité de commande logique (7) ;
- une étape de traitement, dans laquelle ladite unité de commande logique (7) génère une ou plusieurs valeurs de temps (T) sur la base desdits signaux de débit (Q) et calcule un paramètre de filtration (F) du liquide à tester (L) sur la base desdites une ou plusieurs valeurs de temps (T) ;
dans lequel, à ladite étape de détection, lesdits moyens de mesure de débit (6) génèrent chacun desdits signaux de débit (Q) en correspondance avec le passage d'un volume par défaut (V) ;
à ladite étape de traitement, ladite unité de commande logique (7) :

- prend en compte le nombre desdits signaux de débit (Q) arrivant desdits moyens de mesure de débit (6),
- génère l'une desdites une ou plusieurs valeurs de temps (T) en correspondance avec la prise en compte d'un nombre par défaut desdits signaux de débit (Q) ;
- calcule ledit paramètre de filtration (F) sur la base desdites une ou plusieurs valeurs de temps (T) ;

dans lequel ladite unité de commande logique (7) continue à prendre en compte les signaux de débit (Q) jusqu'à l'obtention d'un volume de référence (V') déterminé égal à la somme du nombre des volumes par défaut (V) qui sont passés en correspondance avec chaque signal de débit (Q) pris en compte et calcule ledit paramètre de filtration (F) sur la base de ladite valeur de temps (T) correspondant au passage dudit volume de référence (V') ;
dans lequel ladite unité de commande logique (7) sélectionne la valeur dudit volume de référence (V'), et par conséquent le nombre desdits signaux de débit (Q) à prendre en compte, sur la base dudit paramètre de filtration (F) à calculer ;
ledit procédé étant **caractérisé en ce que** ladite unité de commande logique (7) est pourvue de :

- un module de mémoire (70), dans lequel les formules pour le calcul du paramètre de filtration (F) sont mémorisées ; ledit paramètre de filtration (F) étant l'un ou plusieurs parmi : l'indice de filtrabilité (IF), indice de filtrabilité modifié (IFM), volume maximum filtrable (Vmax) et indice de colmatage (Silt Density Index ou SDI) ;
- une interface utilisateur (71), à travers laquelle un opérateur est susceptible de sélectionner le paramètre de filtration (F) à calculer.

7. Procédé selon la revendication 8, **caractérisé en ce qu'**il prévoit une étape de démarrage, avant ladite étape de traitement, dans laquelle ladite unité de commande logique (7) prend en compte un nombre par défaut initial desdits signaux de débit (Q) et génère une valeur de temps initial (T0) à partir de laquelle ladite étape de traitement a lieu.

**Fig. 1**

```
┌──────────────────────────────────────────────────────────────┐
│                                                                │
│  ┌──────────┐        ┌──────────┐                              │
│  │    6     │───────▶│   74     │          7                   │
│  │  (V, Q)  │        │ (V', V0) │          ─                   │
│  └──────────┘        └──────────┘                              │
│                           │                                    │
│                           ▼                                    │
│  ┌──────────┐        ┌──────────┐                              │
│  │   14     │        │    72    │                              │
│  │   (K)    │──┐      │ (T, T0)  │                              │
│  └──────────┘  │      └──────────┘                             │
│                │           │                                   │
│                │           ▼                                   │
│                │    ┌──────────────┐      F   ┌──────────┐     │
│                │    │      73      │◀───────▶ │    70    │     │
│                └───▶│   (IF, IFM,  │          │          │     │
│                     │  Vmax, SDI)  │          └──────────┘     │
│                     └──────────────┘              ▲ F          │
│                                                   ▼            │
│  ┌──────────┐        ┌──────────┐          ┌──────────┐        │
│  │    13    │        │          │          │    71    │        │
│  │   (P)    │───────▶│    75    │          │          │        │
│  └──────────┘        └──────────┘          └──────────┘        │
│                           │                                    │
└───────────────────────────┼────────────────────────────────────┘
                            ▼
                     ┌──────────┐
                     │    5     │
                     │          │
                     └──────────┘
```

# Fig. 2

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 2019001246 A1

- DE 19831946 A1